# EUROPEAN PATENT APPLICATION

(11) **EP 3 146 935 A1**
(43) Date of publication of application: **29.03.2017**
(21) Application number: 15186594.6
(22) Date of filing: 24.09.2015
(51) Int. Cl.: A61C 13/15, A61B 5/00, A61C 1/08

(54) **A DENTAL LIGHT IRRADIATION DEVICE**

(71) Applicant: 3M Innovative Properties Company, St. Paul, MN 55133-3427 (US)
(72) Inventor: Schepke-Gerlach, Korbinian, 82131 Gauting (DE); Harre, Manfred, 86899 Landsberg am Lech (DE); Bissinger, Peter, 86911 Diessen (DE)
(74) Representative: Hohmann, Arno

(57) **Abstract**

A dental light irradiation device (1) which has a light emitting diode (15) and a heat sink (16) for dissipating heat generated by the LED. The heat sink (16) is formed of a composition which includes a polymer and a filler. The filler provides the composition with increased thermal conductivity relative to the polymer alone. The invention helps maximizing the convenience in handling of dental light devices.

## Description

### Field of the Invention

The invention relates to a dental light irradiation device which comprises a light emitting diode (LED) and a heat sink that is formed of a composition which comprises a polymer and a filler.

### Background Art

In dentistry, light hardenable or light curable dental materials are often used in the treatment or restoration of teeth in a patient's mouth. Dental materials of this type are typically initially liquid or pasty so that they can be easily applied to a desired place, and once the materials are placed, can be hardened by exposing them to light of a certain wavelength or color. Dental materials are typically hardenable by blue light. Typically such light-hardenable dental materials are used for filling cavities in teeth, or for securing dental prostheses to teeth, but they are also used for coating and/or sealing tooth surfaces.

Today many different light devices which are typically used to cure light-hardenable materials are available on the market. Generally such light devices have a powerful light source which provides light of an intensity and wavelength required for hardening the dental materials. Because such a light source typically also generates heat during operation, a light device is typically configured to dissipate the heat from the device, or to cool the device, to avoid overheating of the light source and/or to avoid hot surfaces on the device.

There are for example devices in which the heat of the light source is dissipated by help of air cooling, for example by use of a fan. Such devices typically have venting openings so that the heat can be dissipated by air exchange. There are further devices which do not use air cooling, but which use less heat generating light sources.

For example US 2005/0236586 A1 discloses an irradiation device which comprises a single light-emitting unit and a light-conducting unit. The heat produced by the light-emitting unit during operation of the irradiation device is dissipated to a heat sink which further passes the heat to the appliance housing by thermal conduction, from where it is radiated away to the environment.

Although existing devices may provide a variety of advantages it is still a desire to provide a device having a powerful light source, but which is relatively compact, light-weight, inexpensive, and has low power consumption. An object of the present invention is therefore to provide a light device having at least some advantages over the prior art.

### Summary of the Invention

The invention relates to a dental light irradiation device. The device comprises a light emitting diode (LED) and a heat sink for dissipating heat that is generated by the LED. The heat sink is formed of a composition which comprises a polymer and a filler. The filler provides the composition with increased thermal conductivity of the composition relative to a thermal conductivity of the polymer alone.

The invention is advantageous in that it enables the use of a powerful light source, in particular a LED, in a relatively light weight device. Further, the invention allows a relatively complex design of the heat sink at minimized efforts. A complex design of the heat sink may be desired to achieve a device having minimized dimensions and minimized costs for manufacturing. Further, the invention can be used to facilitate the assembly of the device or parts of the device. The invention also helps minimizing the weight and dimensions of a dental light irradiation device. Thus, the invention helps also maximizing the convenience during handling of the devise.

Preferably the LED is configured to emit blue light. For the purpose of the present specification "blue light" refers to light having a wavelength within a range of about 430 nm (nanometers) and about 490 nm and a peak wavelength within a range of about 444 nm and 453 nm. Further such blue light preferably substantially does not comprise light at wavelengths outside the range of about 430 nm and about 490 nm. For example at least 90%, more preferably 95% of the light quantity emitted from the device is formed by blue light having a wavelength within a range of about 430 nm and about 490 nm.

Although a LED is preferably used with the present invention the heat sink as described herein may be likewise used in combination with other light sources. For example instead or in addition of a LED the device may have a laser light source or another light source as appropriate.

In an embodiment the dental light irradiation device further comprises at least one metal heat dissipation layer. The heat dissipation layer may be arranged between the LED and the heat sink for picking up heat generated by the LED, dissipating the heat within the heat dissipation layer and conducting the heat into the heat sink. The heat dissipation layer may be part of or formed by an electric conductor for electrically powering the LED. The LED may be arranged on one side of a circuit board and the conductor may extend through the circuit board to the opposite side of the circuit board. The circuit board may be attached on the heat sink between the circuit board and the heat sink. Thus, heat generated by the LED flows via the connector toward the heat dissipation layer and into the heat sink.

In one embodiment the heat dissipation layer has a heat sink facing surface which has an area of at least 100 mm² or greater. This provides for relatively quick conduction of heat from the heat dissipation layer into the heat sink. Further the heat dissipation layer has a surface opposite of the heat sink facing surface and a thickness between these two surfaces. The thickness of the heat dissipation layer is preferably between about 0.2 mm to about 1.5 mm. Therefore the thickness is relatively small so that also the heat capacity of the heat dissipation layer is minimized.

In a further embodiment the filler comprises boron nitride. Further fillers like TiO₂, Al₂O₃ and/or graphite may be used. The filler content may be within a range of 10% per weight and 60% per weight, more preferably between 20 % per weight and 40 % per weight, most preferably approximately 30% per weight. The thermal conductivity may be up to 15 W/m K.

In a further embodiment the polymer comprises or is formed of a polypropylene, polyethylene, polyamide, polycarbonate, liquid crystal polymer, polyethylene terephthalate, polyphenylene sulfide, polyphthalamide or polybutadiene terephthalate. In preferred embodiments the polymer is formed of polyamide, polybutadiene terephthalate or polycarbonate.

In a further embodiment the polymer is an epoxy-based resin in which the filler is provided in a range from 20 % per weight and 60% per weight. In this embodiment the filler content can be maximized to up 60% per weight with maintaining acceptable mechanical properties of the heat sin sink.

In one embodiment the composition provides the heat sink with an electric insulating property, the heat sink thus having an electrical resistance of more than 10 000 MΩ.

Therefore the electrical conductors for powering the LED may be arranged directly on the heat sink or spaced by only a layer of adhesive between the conductors and the heat sink.

In a further embodiment the heat sink supports the LED. For example, the heat sink may have means to position and/or retain the LED thereon. Further the heat sink may form at least part of a tubular or ring-shaped reflector. Such a reflector may have a LED-facing conical surface which widens in a direction in which the LED emits light. The LED has typically an angle of radiation (cone beam) and the reflector extends preferably circumferentially about a symmetry axis of the angle of radiation (or cone beam). The angle of the cone may be about 40 and 80 degrees, measured in a cross-sectional plane between opposite sides of the LED-facing surface.

In one embodiment the heat sink forms the reflective surface (which is the LED-facing surface) of the reflector. The reflective surface is preferably not coated or mirrored, although a coated or mirrored surface may be used in some embodiments.

In one embodiment the heat sink houses the LED. For example, at least a part of the heat sink may be formed by an overmold over the LED. Further, at least a portion of the conductor or heat dissipation layer may be embedded in the heat sink (for example overmolded by a portion of the heat sink). Thus, the heat conduction between the LED and/or the conductor/heat dissipation layer may be maximized.

In one embodiment the heat sink forms at least a portion of an outer surface of the device. The heat sink may further form a substantial or predominant portion of the outer surface of the device. Therefore at least a part of a separate housing may not be necessary.

In a further embodiment the heat sink carries and/or embeds components of electric circuitry for controlling the device. Therefore, efforts in the assembly of the electric circuitry may be minimized. In addition this allows the heat sink to be used for cooling the electric circuitry.

In one embodiment the dental light irradiation device comprises a temperature sensor. The sensing portion of the temperature sensor may be embedded within the heat sink. Therefore the temperature of the heat sink may be monitored, and depending on the heat sink temperature the device may be controlled accordingly (for example switched off upon a maximum desired or permissible temperature of the heat sink is detected).

In a further embodiment the dental light irradiation device comprises a body which forms toward a rear end of the device a handle portion of the device. The device further preferably forms a light output tip toward a front end of the device for emitting light generated by the LED. The light output tip is preferably elongated.

In one embodiment the light output tip forms a lighting unit adjacent the front end of the device. The lighting unit may comprise the LED. The lighting unit may further comprise a transparent cover which forms part of a housing of the device and through which light emitted from the LED exits the device.

In a further embodiment the heat sink extends monolithically between the body and the lighting unit. Therefore, heat generated at the front end of the device may be conducted from the relatively narrow light output tip toward the larger body. The portion of the heat sink arranged in the body may be enlarged relative to the portion of the heat sink arranged in the light output tip. Accordingly the portion of the heat sink arranged in the body may be have a greater heat capacity than the portion of the heat sink arranged in the light output.

### Brief Description of the Figures

- Fig. 1: is a cross-sectional view of a dental light irradiation device according to an embodiment of the invention;
- Fig. 2: is a detail view relating to a dental light irradiation device according to an embodiment of the invention;
- Fig. 3: is a perspective view of a heat sink as it may be used with a dental light irradiation device according to an embodiment of the invention; and
- Fig. 4: is a cross-sectional view of a further heat sink and an LED module as they may be used with a dental light irradiation device according to another embodiment of the invention.

### Detailed Description of the Invention

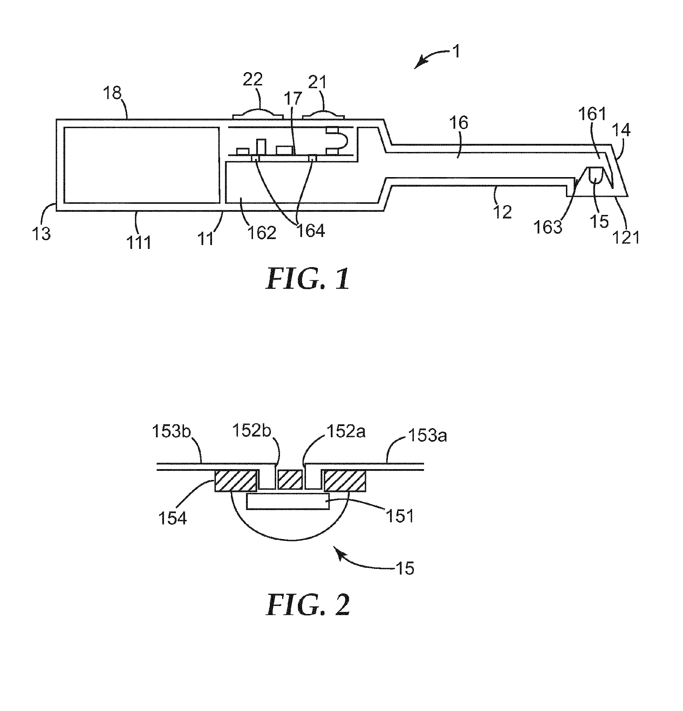

Fig. 1 shows an example of a dental light irradiation device 1 according to the invention. The device 1 has a body 11 and an elongated light output tip 12. The body 11 extends toward a rear end 13 of the device 1 and further forms a handle portion 111. The handle portion 111 allows holding of the device 1 by a user. The light output tip 12 extends toward a front end 14 of the device. The light output tip 12 is shaped and dimensioned so that it can enter a patient's mouth for example for light hardening a dental filling material in one or more of the patient's teeth. The light output tip 12 has a light output 121 for emitting light which is generated by an LED module 15. The LED module 15 in the example comprises at least one high power LED. In particular the LED module 15 is configured for emitting blue light as specified herein.

The device 1 has a heat sink 16 formed of a composition which comprises a polymer and a filler. The filler provides the composition with increased thermal conductivity compared to the same polymer without the filler. In the example the heat sink 16 extends between the light output tip 12 and the body 11. In particular, the heat sink 16 has a front end 161 which is arranged adjacent the front end 14 of the device 1 and rear end 162 arranged within the body 11. A portion of the heat sink 16 at the front end 161 carries the LED module 15 and further forms a reflector 163 which extends circumferentially around the LED module 15. The reflector is cone-shaped and has a cone, angle of about 50 degrees, widening toward a direction in which the LED module 15 (or the LED) emits light. The angle is measured in a plane through the axis of symmetry of the cone between opposite sides of the reflective surface. The reflector 163 in the example is not coated or mirrored but is provided with the reflective properties by the composition from which the heat sink 16 is made. A further portion of the heat sink 16 at the rear end 162 is configured for carrying electric circuitry 17 for controlling the device 1. The heat sink 16 therefore has means 164 for mechanically retaining the electric circuitry 17 on the heat sink. Such means may comprise pins, screw-holes, retainers and/or other structures, as appropriate.

The device 1 further has a housing 18 which encapsulates the heat sink 16 with the electric circuitry 17 and the LED module 15. The heat sink 16 is circumferentially spaced from the housing, except for some spacers (not illustrated) which hold the heat sink 16 in place relative to the housing 18. The housing 18 may be generally made of metal except for the light output 121 which is made of a light transmissible (for example transparent) material, like glass or plastic.

The device 1 in this example further comprises a first knob 21 and a second knob 22. The first knob 21 is an activator for switching the device 1 on or off. The second knob 22 is a selector for pre-selecting an operating time period for which the device 1 operates before it switches off automatically. For the purpose of the present specification switching the device 1 on causes the device 1 to operate and an operation causes the device to emit light via the light output. Further switching the device 1 off causes the device 1 the device to suspend the emission of light via the light output. The device 1 has further functionality which is independent or at least not directly dependent on the operation of the device. The device has, for example, a battery 19, which may be charged or chargeable independent from any operation of the device 1.

Fig. 2 shows the LED module 15 in more detail. The LED module 15 in this example has a LED semiconductor 151 which is bonded to two conductors 152a, 152b. The LED semiconductor 151 is covered by a transparent lens 155. The lens 155 is preferably formed by an overmold over the semiconductor. Such an overmold may be made of a hardened transparent resin, for example. The conductors 152a, 152b provide for electrically connecting the LED semiconductor 151 with a power source. The conductors 152a, 152b are each configured to comprise or form a heat dissipation layer 153a, 153b. Each heat dissipation layer 153a, 153b is formed by a sheet-like section that is formed by the respective conductor 152a, 152b. Such sheet-like section is relatively flat and forms at least one relatively large major surface. For example, the sheet-like section may be widened with respect to adjacent sections of the conductor in one dimension laterally of a dimension along which the conductor extends. The size in the third dimension of the sheet-like layer may correspond to or may be smaller than the size of the conductor in the same dimension.

The heat dissipation layers 153a, 153b are arranged with their major surface in contact with the heat sink 16. For example, the heat dissipation layers 153a, 153b may be embedded within the heat sink 16 (not visible in this view). Such an embedding may be achieved by molding the heat sink 16 over heat dissipation layers 153a, 153b. Thus, heat conducted from the LED semiconductor 151 can be transferred by conduction via the relatively large major surface into the heat sink 16. Therefore, the heat can be conveyed into the heat sink 16 relatively rapidly. Further, because the heat dissipation layer 153a, 153b is relatively flat, the heat capacity of the heat dissipation layer 153a, 153b is relatively low so that a relatively low amount of heat is captured in the heat dissipation layers 153a, 153b. The conductors 152a, 152b may be configured as a lead frame or may be part of a circuit board. A circuit board 154 is shown in the example.

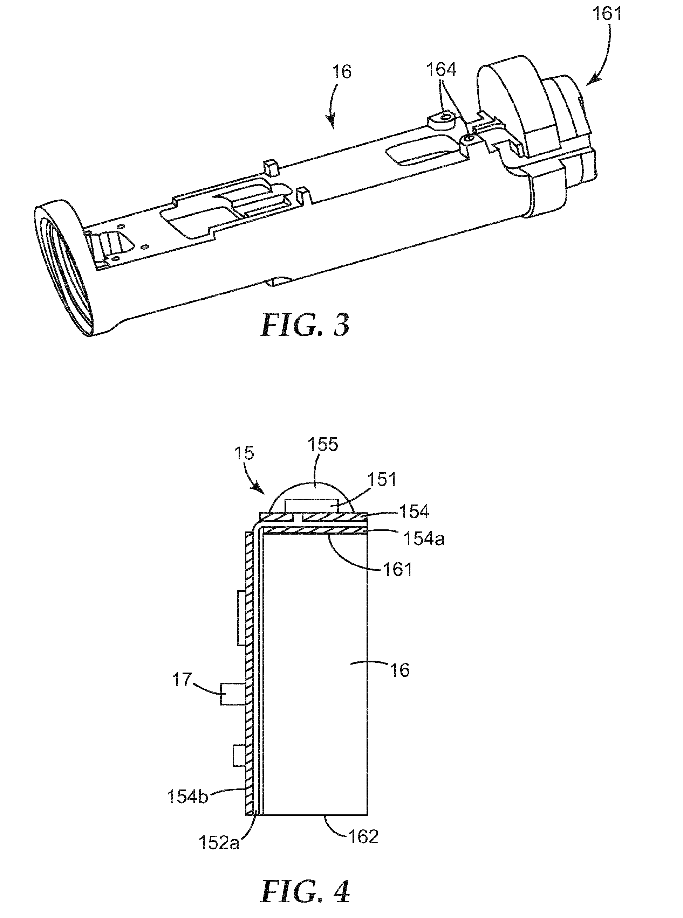

Fig. 3 shows an alternative heat sink 16 that is formed of a composition which comprises a polymer and a filler providing the composition with increased thermal conductivity relative to the polymer alone. The front end 161 of this heat sink 16 is configured to cooperate with a light guide (not shown) that guides light generated at the front end 161 toward a light output. Such a light device is for example disclosed in WO 2010/068435 A1. In this example the LED module (not shown) is arranged on the front end 161 of the heat sink 16. Therefore a portion of the heat sink 16 at the front end 161 may form a reflector (not illustrated) which extends circumferentially around the LED module as described above. The heat sink 16 has a portion at the rear end 162 of the heat sink for carrying electric circuitry (not illustrated) for controlling the device 1. The heat sink 16 therefore has means 164 for mechanically retaining the electric circuitry 17 on the heat sink. Such means in the example comprise screw-holes.

Fig. 4 shows a further alternative heat sink 16 that is formed of a composition which comprises a polymer and a filler providing the composition with increased thermal conductivity relative to the polymer alone. In this embodiment the LED module 15 has an angled circuit board 154 forming a first portion 154a and a second portion 154b. At least one of the conductors 152a of the LED module 151 continues between the first and second portion 154a, 154b. The LED module 151 is arranged on the first portion 154a of the circuit board 154 and electrically connected to two conductors (of which only conductor 152a is visible in this view). The second portion 154b forms electric circuitry 17 for controlling the device of the invention. The conductor 152a forms a heat dissipation layer at least within a portion of the second portion 154b as described in the example of Fig. 2. Further also the further conductorforms a heat dissipation layer at least within a portion of the second portion 154b as described in the example of Fig. 2. For example, the two heat dissipation layers may be arranged side by side over the full length of the heat sink 16. The length of the heat sink is the dimension of the heat sink 16 between the front end 161 and the back end 162 of the heat sink 16. The heat sink 16 as illustrated in this example may be used in combination with a light guide (not shown) as described in the example of Fig. 3.

## Claims

1. A dental light irradiation device, comprising a light emitting diode (LED) and a heat sink for dissipating heat that is generated by the LED, the heat sink being formed of a composition which comprises a polymer and a filler which provides the composition with increased thermal conductivity relative to the polymer alone.

2. The dental light irradiation device of claim 1, further comprising at least one metal heat dissipation layer which is arranged between the LED and the heat sink for picking up heat generated by the LED, dissipating the heat within the heat dissipation layer and conducting the heat into the heat sink.

3. The dental light irradiation device of claim 1 or 2, wherein the heat dissipation layer is formed by an electric conductor for electrically powering the LED.

4. The dental light irradiation device of any of the preceding claims, wherein the filler comprises boron nitride.

5. The dental light irradiation device of any of the preceding claims, wherein the filler content is within a range of 10% per weight and 60% per weight.

6. The dental light irradiation device of any of the preceding claims, wherein the polymer comprises or is formed of a polypropylene, polyethylene, polyamide, polycarbonate, liquid crystal polymer, polyethylene terephthalate, polyphenylene sulfide, polyphthalamide or polybutadiene terephthalate.

7. The dental light irradiation device of any of the preceding claims, wherein the composition provides the heat sink with an electric insulating property, the heat sink thus having an electrical resistance of more than 10 000 MΩ.

8. The dental light irradiation device of any of the preceding claims, wherein the heat sink supports the LED and forms at least part of a tubular reflector, the LED having an angle of radiation, and wherein the reflector extends circumferentially about a symmetry axis of the angle of radiation.

9. The dental light irradiation device of claim 8, wherein the heat sink forms the reflective surface of the reflector.

10. The dental light irradiation device of any of the preceding claims, wherein the heat sink forms at least a portion of an outer surface of the device.

11. The dental light irradiation device of any of the preceding claims, wherein the heat sink carries and/or embeds components of electric circuitry for controlling the device.

12. The dental light irradiation device of any of the preceding claims, comprising a temperature sensor which sensing portion is embedded within the heat sink.

13. The dental light irradiation device of any of the preceding claims, comprising a body which forms toward a rear end of the device a handle portion of the device, and an elongated light output tip toward a front end of the device for emitting light generated by the LED.

14. The dental light irradiation device of claim 13, wherein the light output tip forms a lighting unit adjacent the front end of the device, and wherein the lighting unit comprises the LED.

15. The dental light irradiation device of claim 14, wherein the heat sink extends monolithically between the body and the lighting unit.
